# EUROPEAN PATENT SPECIFICATION

(11) **EP 3 062 689 B1**
(45) Date of publication and mention of the grant of the patent: **12.07.2023**
(21) Application number: 14857711.7
(22) Date of filing: 03.03.2014
(51) Int. Cl.: A61B 5/389, A61B 5/00

(54) **SYSTEM FOR DETERMINING IF SURGICALLY-IMPLANTED HARDWARE SHOULD BE REMOVED**
SYSTEM ZUR BESTIMMUNG, OB EINE CHIRURGISCH IMPLANTIERTE HARDWARE ENTFERNT WERDEN SOLLTE
SYSTÈME POUR DÉTERMINER LE BESOIN DE RETIRER UN MATÉRIEL IMPLANTÉ CHIRURGICALEMENT

(30) Priority: 01.11.2013 US 201314069728
(43) Date of publication of application: 07.09.2016
(73) Proprietor: Reaston, Maryrose Cusimano, Tulsa, OK 74133 (US); Tomecek, Frank J. Junior, Tulsa, OK 74132 (US); Reaston, Phil, Tulsa, OK 74133 (US)
(72) Inventor: Reaston, Maryrose Cusimano, Tulsa, OK 74133 (US); Tomecek, Frank J. Junior, Tulsa, OK 74132 (US); Reaston, Phil, Tulsa, OK 74133 (US)
(74) Representative: Cabinet Chaillot
(86) International application number: PCT/US2014/019827
(87) International publication number: WO 2015/065512

(56) References cited:
- US-A1- 2006 052 782
- US-A1- 2007 287 900
- US-A1- 2008 294 258
- US-A1- 2009 299 210
- US-A1- 2010 292 963
- US-A1- 2011 201 904
- US-A1- 2011 224 503

## Description

### TECHNICAL FIELD

The invention generally pertains to surgically-implanted hardware, and more particularly to a system for determining if surgically-implanted cervical, thoracic or lumbar hardware should be removed.

### BACKGROUND ART

Stabilization and fusion of a person's lumbar spine may be performed by using various anterior and posterior surgical techniques and a wide range of hardware, including screws, spinal wires, and vertebral cages. The hardware is designed to stabilize the spine after fusion. Post operative imagining is typically used to determine the progress and positioning of the hardware. The type of imaging depends on the type of hardware. Currently, there is no reference standard for non-invasive imaging evaluation of fusion.

Radiography is the noninvasive modality most commonly used for the assessment of fusion. Radiology can have limited benefits, especially if breakage of the hardware or incorrect placement of the hardware is suspected. Flexion and extension views have been recommended for routine assessment of hardware post fusion, but there is no objective clinical data to support this use for the routine assessment of fusion.

A computer tomography (CT) scan is another modality for imaging bony detail in a person's spine during post surgical evaluation. However, the quality of CT images may be degraded because of artifacts due to metallic implants. While magnetic resonance imaging (MRI) also has artifacts, the artifacts seen on CT are not limited to the area immediately adjacent to the surgical area. Also with both CT and MRI, movement artifact(s) also significantly affect evaluation of most surgical changes.

MRI is one of the better imaging tools to evaluate postoperative changes in the spine, but it is much more sensitive to detect soft tissue and neuro-vascular changes than it is in detecting bone and arthrodesis. It is particularly useful for detecting and monitoring infection or postoperative scar. However, magnetic susceptibility artifact(s) may be a problem, particularly in the presence of stainless steel devices. Recent hardware is made of titanium alloys and even though they produce less severe magnetic artifacts, the artifacts still present a significant obstacle to visualization of areas in close proximity to the hardware.

In order for any kind of imaging to be beneficial, pre and post-operative comparisons are necessary. Other techniques such as ultrasonography, bone scans, and myelography have little or no benefit in assessing post-operative hardware.

The primary types of hardware most commonly used in spinal fusion are: interbody spacers, plates or rods with pedicle screws and translaminar or facet screws. Unfortunately, the hardware may need to be removed because of infection, damage or pain. A significant issue facing surgeons is trying to distinguish patients who have hardware failures, loosening or pseudoarthrosis, from patients who have pain due to hardware irritating the paraspinal soft tissue. Currently, a method of assessing if hardware removal will be beneficial is the injection of a local anesthetic around the site of the pain. The patient is asked to show their doctor the point of tenderness, an injection (typically lidocaine) is injected at the site of tenderness and then the patient is asked if the pain is better. If they report a decrease in pain then if might be that the hardware is contributing to the pain. This is the current method is use to determine if hardware removal will be beneficial. This method is not objective, fraught with subjectivity and does not confirm to the guidelines of evidence based medicine. This is not only problematic but may subject the patent to unnecessary surgery to remove the hardware.

Hardware removal is the most common elective orthopedic procedure, accounting for 30-50% of all planned orthopedic surgical procedures. The surgeries not only require a second surgical procedure in tissue that is already scarred but also poses a risk for nerve injury and recurrent spinal deformity or delayed fusion failures. It is essential that surgeons recognize lumbar hardware removal as a major surgery. Most series report a complication rate ranging from 3% to 20%. Complications include infection and bleeding, which is sometimes a challenge to control due to large bony defects after pedicle screw removal. Additionally, hardware removal may alleviate but not fully correct the problem; and retained hardware may be only one of several pain generators in the post-operative spine. Pain is the most predominant indication for removal of the hardware. Doctors have even named this condition "painful hardware syndrome", yet there is no diagnostic code that reflects such as a syndrome. As a result, there is an overwhelming need for an objective method to evaluate if hardware needs to be removed after surgery.

A case study from the Texas Scottish Rite Hospital for Children reviewed results after implanted hardware removal in. scoliosis patients. Unfortunately, 8% of the patients who underwent hardware removal required reoperation due to pain; and 5% of the patients required reoperation due to infection. Overall, after the removal of the hardware, there was an immediate progression of 3% in the thoracic and 6% in the lumbar scoliotic curves and sagittal plane progression including kyphosis. Instead of alleviating the problem, the removal of the hardware worsened the patient's curvature and sagittal balance. Therefore, it is essential, especially in long fusions for scoliosis, that the physician and patient are fully confident that the patient will be an appropriate candidate hardware removal. in the case of scoliosis patients, even those with a solid fusion, hardware removal may not be in their best interest; the potential benefit should outweigh the risks to the patient in this type of procedure.

A search of the prior art did not disclose any literature or patents that read directly on the claims of the instant invention. However, the following U.S. patents are considered related:

| **PATENT NO.** | **INVENTOR** | **ISSUED** |
|---|---|---|
| 4,887,595 | Helnig | 19 Dec. 1989 |
| 5,055,104 | Ray | 8 Oct. 1991 |

US 4,887,595 patent discloses a surgically implantable device for maintaining the relative positions of spinal bodies of a spinal column. The device comprises a plate for connection with a first spinal body. The plate portion has a first side surface for facing the first spinal body and a second side surface. The plate portion has an opening which extends through the first and second side surfaces for receiving a fastener to connect the plate portion with the first spinal body.

US 5,055,104 patent discloses a fusion cage that is utilized when an intervertebral fusion is requested. The fusion cage preserve the disc space and the implanted by an anterior material to the lower back. The cage can be removed after the vertebrae have become fused together and without disrupting that fusion, thus guarding against possible rejection of the cages by the patient's body.

For background purposes and indicative of the art to which the invention relates, reference may be made to the following remaining US patents found in the patent search.

| **PATENT NO.** | **INVENTOR** | **ISSUED** |
|---|---|---|
| 4,157,085 | Austad | 5 June 1979 |
| 5,284,130 | Ratliff | 8 Feb. 1994 |
| 5,607,480 | Beaty | 4 March 1997 |
| 5,816,811 | Beaty | 6 Oct. 1998 |
| 5,954,638 | Spranza, III | 21 Sep. 1999 |
| 8,409,075 | Chu | 2 Apr. 2013 |

### DISCLOSURE OF THE INVENTION

The invention is defined by appended claims 1-7.

The invention relates to a system comprising an electrodiagnostics functional assessment (EFA) device functioning in tandem with a computer device operating proprietary software for medical evaluation and multiple wireless leads attached to an individual's back adjacent a site of cervical, thoracic or lumbar hardware implantation, wherein the electrodiagnostics functional assessment (EFA) device is configured by the computer device to perform a test protocol in order to acquire data, wherein said data is selected from the group consisting of electromyography (EMG), range-of-motion (ROM), functional capacity assessment (FCA), muscle data, myofascial data, structural data, nerve data or ischemic data, wherein said test protocol comprises the steps:
a) select an individual who is a candidate for removal of surgically-implanted hardware,
b) determine with the EFA device functioning in tandem with the computer device and the multiple wireless leads attached to the individual's back adjacent the site of hardware implantation if the hardware removal has organic or objective etiology,
c) prepare said EFA device for selected test protocol with the computer device functioning in tandem with the EFA device and operating proprietary software for medical evaluation,
d) perform at least one rest and return to rest test protocol for EMG, if selected,
e) perform at least one rest and return to rest test for ROM, if selected,
f) perform at least one rest and return to rest test for FCA, if selected,
g) acquire test protocol results and data wirelessly sent from the multiple wireless leads attached to the individual's back adjacent the site of hardware implantation to the EFA device wherein the data will disclose if muscle, ischemic or nerve damage is present in the area of the surgically-implanted hardware. Preferred embodiments are defined in the dependent claims.

### BRIEF DESCRIPION OF THE DRAWINGS

FIGURE 1 is a flow diagram showing each of the steps that are utilized in the test protocol.
FIGURE 2 is an elevational view showing an individual undergoing a test protocol from an EFA device. Multiple wireless leads are attached to the individual's back adjacent the site of hardware implantation. Diagnostic data is wirelessly sent from the leads to the EFA for diagnosis.

### BEST MODE FOR CARRYING OUT THE INVENTION

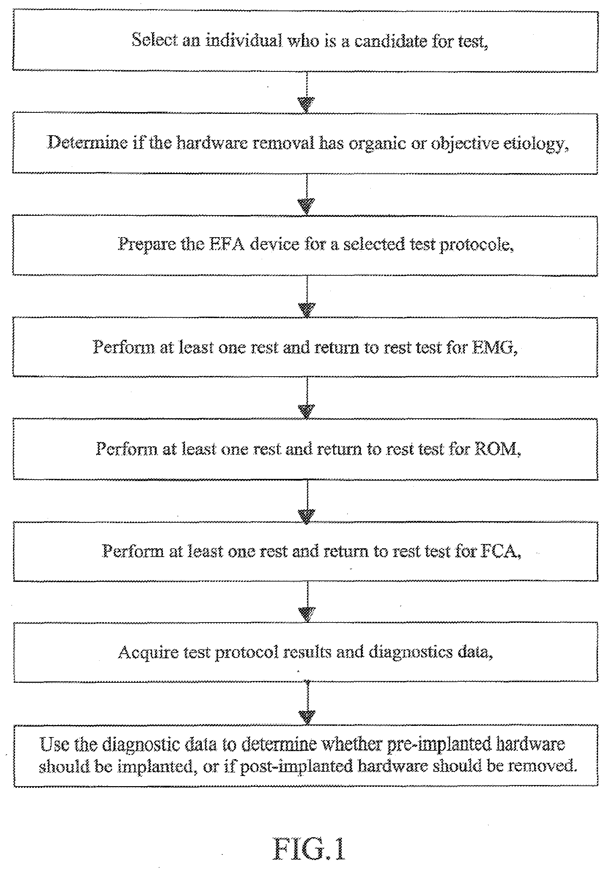

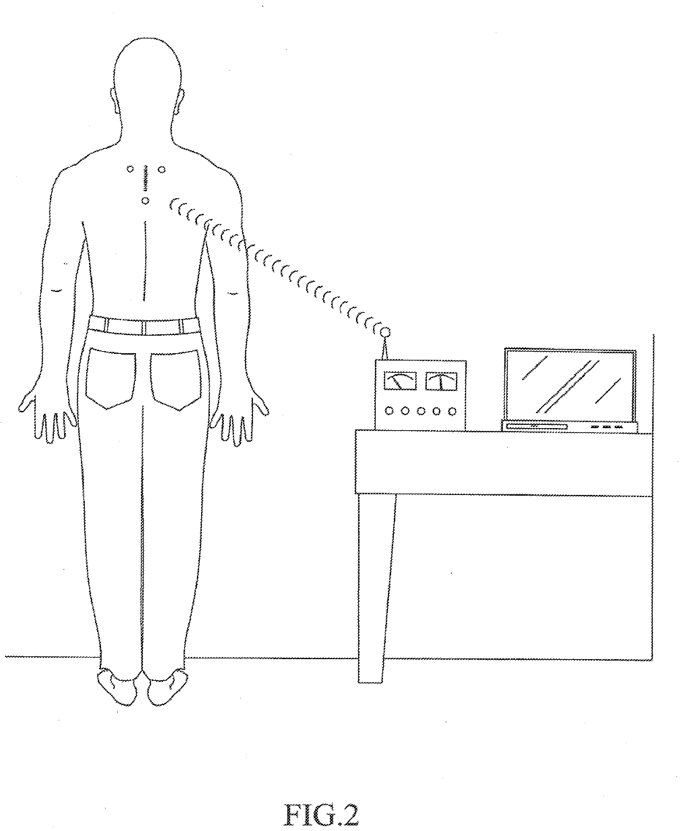

The best mode for carrying out the invention is presented in terms that disclose a system for determining if surgically-implanted hardware should be removed from a person. The operation of the system as disclosed in steps, described and shown in FIGURES 1-2, provides an effective means for healthcare professionals to determine if surgically-implanted hardware must be removed, should be removed or should remain implanted. The system is configured to analyze diagnostic data acquired from a test protocol from an Electrodiagnostic Functional Assessment device (EFA). There are currently two main versions of the EFA: the EFA-2 which utilizes wired technology, and the EFA-3 which utilized wireless technology. Both the EFA-2 and the EFA-3 are issued U.S. patents to the applicant (EFA-2 patent no. 8,535,224 and EFA-3 patent no. 8,568,312).

Regardless of which version is employed, the EFA is utilized to acquire and provide the diagnostic data that is based on the testing protocol to determine if surgically-implanted hardware should be removed. The diagnostic data discloses the information that indicates whether hardware removal is appropriate. The method is conducted in the event there is no infection, or broken or dislodged hardware in the person, of if there are no other objective findings disclosed from other diagnostic tests, such as x-rays, CT scans or MRI.

The EFA simultaneously monitors electromyography, range-of-motion and functional capacity assessment. The muscle groups monitored for the instant method are paraspinal, cervical, quadratus lumborum, gluteal, upper and lower extremities and muscles in the sacro iliac region. The EFA functions in tandem with a computer having a touch screen or keyboard and running proprietary software which correlates muscle activity, ischemic activity with the electromyography, range-of-motion, and a functional capacity assessment.

The initial process for utilizing the instant method 10 is to perform a pre-surgical baseline EFA test protocol. A post surgical evaluation is then done after substantially 3-months post surgery for comparison to the pre-surgical baseline and as a reference should there be a potential hardware problem(s). The EFA test protocol is then repeated and compared to the prior baseline test protocol. In the event of potential hardware problem(s) a pre-surgical and post surgical after 3-month baseline test protocol is not required. The method does require an EFA assessment at the time of the consult for potential hardware removal. The method will assess if there is a problem(s) resulting from failed surgery, a hardware problem or a myofascial problem, and be able to identify the issue and prevent unnecessary surgery.

The test protocols for the method include but are not limited to rest and return to rest readings, ROM readings and FCA for the specific body parts evaluated.
a) select an individual who is a candidate for either pre-surgical hardware implantation, post-surgical hardware implantation or pre-removal of surgically-implanted hardware,
b) determine if the hardware removal has organic or objective etiology,
c) prepare said EFA device for selected test protocol,
d) perform at least one rest and return to rest test protocol for EMG, if selected,
e) perform at least one rest and return to rest test for ROM, if selected,
f) perform at least one rest and return to rest test for FCA, if selected,
g) acquire test protocol results and diagnostic data, wherein the diagnosis data will disclose if muscle, ischemic or nerve damage is present in the area of the surgically-implanted hardware, and
h) use the diagnostic data to determine whether pre-implanted hardware should be implanted, or if post-implanted hardware should be removed.

Data will not only yield specific information about muscles, nerves, spinal changes and ischemic changes, but will yield information about effort which can be an indication of symptom magnification and could be an indicator that hardware removal is not necessary. The test protocol for the method also includes data from the paraspinal muscles and surrounding musculature to identify if the data correlates to patient complaints and hardware location. The test protocol is performed bilaterally as often a hardware problem(s) will be present unilaterally. The method indicates if the diagnostic data discloses no ischemic changes or nerve changes in the paraspinal muscle which indicates there is no need for hardware removal. If the surrounding musculature has changes not consistent with complaints, the method also indicates that hardware removal is not required. The method does indicate if muscle, ischemic and or nerve data is present in the area of complaint, and this a good indication that hardware removal is required. The method also utilizes the EMG, ROM and FCA data in multiple related combinations with flexion, extension and rotation; lifting, pulling and pushing; and spinal range of motion.

The invention is defined by the appended claims 1-7.

## Claims

1. A system comprising an electrodiagnostics functional assessment (EFA) device functioning in tandem with a computer device operating proprietary software for medical evaluation and multiple wireless leads attached to an individual's back adjacent a site of cervical, thoracic or lumbar hardware implantation, wherein the electrodiagnostics functional assessment (EFA) device is configured by the computer device to perform a test protocol in order to acquire data, wherein said data is selected from the group consisting of electromyography (EMG), range-of-motion (ROM), functional capacity assessment (FCA), muscle data, myofascial data, structural data, nerve data or ischemic data, wherein said test protocol comprises the steps:
a) select an individual who is a candidate for removal of surgically-implanted hardware,
b) determine with the EFA device functioning in tandem with the computer device and the multiple wireless leads attached to the individual's back adjacent the site of hardware implantation if the hardware removal has organic or objective etiology,
c) prepare said EFA device for selected test protocol with the computer device functioning in tandem with the EFA device and operating proprietary software for medical evaluation,
d) perform at least one rest and return to rest test protocol for EMG, if selected,
e) perform at least one rest and return to rest test for ROM, if selected,
f) perform at least one rest and return to rest test for FCA, if selected,
g) acquire test protocol results and data wirelessly sent from the multiple wireless leads attached to the individual's back adjacent the site of hardware implantation to the EFA device wherein the data will disclose if muscle, ischemic or nerve damage is present in the area of the surgically-implanted hardware.

2. The system as specified in claim 1 wherein said EMG data is evaluated bilaterally with ROM data or FCA data.

3. The system as specified in claim 1 wherein the ROM data is selected from the group consisting of flexion, extension and rotation, in combination with EMG data or FCA data.

4. The system as specified in claim 1 wherein the FCA data is selected from the group consisting of lifting, pulling and pushing, in combination with ROM data or EMG data.

5. The system as specified in claim 1 wherein said EFA data is acquired post-surgical implantation of the hardware, or prior to pending hardware removal.

6. The system as specified in claim 1 wherein the test protocol further comprises data acquired from paraspinal muscles and muscles surrounding the paraspinal region to determine if said data correlates to the location of the surgically-implanted hardware.

7. The system as specified in claim 1 wherein said EFA monitors muscles, comprising paraspinal muscles, cervical muscles, upper extremity muscles, lower extremity muscles, gluteal muscles, hip muscles or sacro iliac muscles.

## Patentansprüche

1. - System, umfassend eine Elektrodiagnostik-Funktionsbewertungs (EFA)-Vorrichtung, die zusammen mit einer Computervorrichtung arbeitet, die anwendereigene Software zur medizinischen Bewertung und zahlreiche drahtlose Leitungen betreibt, die an den Rücken eines Individuums benachbart einer Position einer zervikalen, thorakalen oder lumbaren Hardware-Implantation befestigt sind, wobei die Elektrodiagnostik-Funktionsbewertungs (EFA)-Vorrichtung durch die Computervorrichtung konfiguriert ist, um ein Prüfprotokoll durchzuführen, um Daten zu erfassen, wobei die Daten ausgewählt sind aus der Gruppe, bestehend aus Elektromyographie (EMG), Bewegungsumfang (ROM), Funktionsfähigkeitsbewertung (FCA), Muskeldaten, myofaszialen Daten, strukturellen Daten, Nervendaten oder ischämischen Daten, wobei das Prüfprotokoll die folgenden Schritte umfasst:
a) Auswählen eines Individuums, das Kandidat für die Entfernung von chirurgisch implantierter Hardware ist,
b) Bestimmen, mit der EFA-Vorrichtung, die zusammen mit der Computervorrichtung und den zahlreichen drahtlosen Leitungen arbeitet, die an den Rücken des Individuums benachbart Position einer Hardware-Implantation befestigt sind, ob dir Entfernung von Hardware eine organische oder eine objektive Ätiologie aufweist,
c) Vorbereiten der EFA-Vorrichtung für ein ausgewähltes Prüfprotokoll, wobei die Computervorrichtung zusammen mit der EFA-Vorrichtung arbeitet und anwendereigene Software zur medizinischen Bewertung betreibt,
d) Durchführen mindestens einer Pause und Zurückkehren zum Pausenprüfprotokoll für EMG, falls ausgewählt,
e) Durchführen mindestens einer Pause und Zurückkehren zum Pausenprüfprotokoll für ROM, falls ausgewählt,
f) Durchführen mindestens einer Pause und Zurückkehren zum Pausenprüfprotokoll für FCA, falls ausgewählt,
g) Erfassen von Prüfprotokollergebnissen und Daten, die drahtlos von den zahlreichen drahtlosen Leitungen gesendet werden, die an den Rücken des Individuums benachbart der Position einer Hardware-Implantation an die EFA-Vorrichtung befestigt sind, wobei die Daten offenbaren, ob eine Muskel-, ischämische oder Nervenbeschädigung im Bereich der chirurgisch implantierten Hardware vorliegt.

2. - System nach Anspruch 1, wobei die EMG-Daten bilateral mit ROM-Daten oder FCA-Daten bewertet werden.

3. - System nach Anspruch 1, wobei die ROM-Daten ausgewählt sind aus der Gruppe, bestehend aus Flexion, Extension und Rotation in Kombination mit EMG-Daten oder FCA-Daten.

4. - System nach Anspruch 1, wobei die FCA-Daten ausgewählt sind aus der Gruppe, bestehend aus Heben, Ziehen und Schieben in Kombination mit ROM-Daten oder EMG-Daten.

5. - System nach Anspruch 1, wobei die EFA-Daten nach der chirurgischen Implantation der Hardware oder vor der ausstehenden Entfernung der Hardware erfasst werden.

6. - System nach Anspruch 1, wobei das Prüfprotokoll weiter Daten umfasst, die von paraspinalen Muskeln und Muskeln erfasst werden, die die paraspinale Region umgeben, um zu bestimmen, ob die Daten mit der Stelle der chirurgisch implantierten Hardware korrelieren.

7. - System nach Anspruch 1, wobei die EFA Muskeln überwacht, umfassend paraspinale Muskeln, zervikale Muskeln, Muskeln der oberen Extremität, Muskeln der unteren Extremität, Gesäßmuskeln, Hüftmuskeln oder Iliosakralmuskeln.

## Revendications

1. - Système comprenant un dispositif d'évaluation fonctionnelle d'électrodiagnostic (EFA) fonctionnant en tandem avec un dispositif informatique exploitant un logiciel propriétaire pour évaluation médicale et de multiples dérivations sans fil fixées au dos d'un individu de manière adjacente à un site d'implantation de matériel cervical, thoracique ou lombaire, le dispositif d'évaluation fonctionnelle d'électrodiagnostic (EFA) étant configuré par le dispositif informatique pour exécuter un protocole de test afin d'acquérir des données, lesdites données étant sélectionnées dans le groupe constitué d'une électromyographie (EMG), d'une amplitude du mouvement (ROM), d'une évaluation de capacité fonctionnelle (FCA), de données musculaires, de données myofasciales, de données structurelles, de données nerveuses ou de données ischémiques, ledit protocole de test comprenant les étapes :
a) sélectionner un individu qui est un candidat pour le retrait d'un matériel implanté chirurgicalement,
b) déterminer, avec le dispositif EFA fonctionnant en tandem avec le dispositif informatique et les multiples dérivations sans fil fixées au dos de l'individu de manière adjacente au site d'implantation du matériel, si le retrait de matériel a une étiologie organique ou objective,
c) préparer ledit dispositif EFA pour le protocole de test sélectionné, avec le dispositif informatique fonctionnant en tandem avec le dispositif EFA et exploitant un logiciel propriétaire pour évaluation médicale,
d) effectuer au moins un protocole de test au repos et de retour au repos pour EMG, si elle est sélectionnée,
e) effectuer au moins un test au repos et de retour au repos pour ROM, si elle est sélectionnée,
f) effectuer au moins un test au repos et de retour au repos pour FCA, si elle est sélectionnée,
g) acquérir des résultats de protocole de test et des données envoyées sans fil depuis les multiples dérivations sans fil fixées au dos de l'individu de manière adjacente au site d'implantation de matériel au dispositif EFA, dans lequel les données révéleront si des dommages musculaires, ischémiques ou nerveux sont présents dans la zone du matériel implanté chirurgicalement.

2. - Système selon la revendication 1, dans lequel lesdites données EMG sont évaluées bilatéralement avec des données ROM ou des données FCA.

3. - Système selon la revendication 1, dans lequel les données ROM sont sélectionnées parmi le groupe constitué d'une flexion, d'une extension et d'une rotation, en combinaison avec des données EMG ou des données FCA.

4. - Système selon la revendication 1, dans lequel les données FCA sont sélectionnées parmi le groupe constitué d'un soulèvement, d'une traction et d'une poussée, en combinaison avec des données ROM ou des données EMG.

5. - Système selon la revendication 1, dans lequel lesdites données EFA sont acquises après implantation chirurgicale du matériel, ou avant le retrait en attente du matériel.

6. - Système selon la revendication 1, dans lequel le protocole de test comprend en outre des données acquises à partir de muscles paraspinaux et de muscles entourant la région paraspinale pour déterminer si lesdites données sont en corrélation avec l'emplacement du matériel implanté chirurgicalement.

7. - Système selon la revendication 1, dans lequel ledit EFA surveille des muscles, comprenant des muscles paraspinaux, des muscles cervicaux, des muscles des extrémités supérieures, des muscles des extrémités inférieures, des muscles fessiers, des muscles de la hanche ou des muscles sacro-iliaques.
